# EUROPÄISCHE PATENTSCHRIFT

(11) **EP 3 773 311 B1**
(45) Veröffentlichungstag und Bekanntmachung des Hinweises auf die Patenterteilung: **18.10.2023**
(21) Anmeldenummer: 19716092.2
(22) Anmeldetag: 26.03.2019
(51) Int. Cl.: A61B 46/10, A61B 46/17

(54) **MONTAGEADAPTER ZUR BEFESTIGUNG EINES STERILÜBERZUGS AN EINEM MIKROSKOP, MIKROSKOP ZUR VERWENDUNG MIT EINEM DERARTIGEN ADAPTER SOWIE SYSTEM MIT EINEM DERARTIGEN MIKROSKOP UND EINEM DERARTIGEN ADAPTER**
MOUTING ADAPTER FOR SECURING A STERILE COVER ON A MICROSCOPE, MICROSCOPE FOR USE WITH AN ADAPTER OF THIS TYPE, AND SYSTEM HAVING A MICROSCOPE OF THIS TYPE AND AN ADAPTER OF THIS TYPE
ADAPTATEUR DE MONTAGE POUR LA FIXATION D'UNE HOUSSE STÉRILE À UN MICROSCOPE, MICROSCOPE UTILISABLE AVEC UN TEL ADAPTATEUR ET SYSTÈME POURVU D'UN TEL MICROSCOPE ET D'UN TEL ADAPTATEUR

(30) Priorität: 28.03.2018 DE 102018107357
(43) Veröffentlichungstag der Anmeldung: 17.02.2021
(73) Patentinhaber: Digital Surgery Systems, Inc., Goleta, CA 93117 (US)
(72) Erfinder: SCHOLTEN, Thomas, 78532 Tuttlingen (DE); STEINHILPER, Klaus-Dieter, 78532 Tuttlingen (DE); DELLE, Marc, 78628 Rottweil (DE)
(74) Vertreter: K&L Gates LLP
(86) Internationale Anmeldenummer: PCT/EP2019/057611
(87) Internationale Veröffentlichungsnummer: WO 2019/185640

(56) Entgegenhaltungen:
- EP-A1- 3 178 438
- EP-A1- 3 178 438
- WO-A2-2009/037193
- WO-A2-2009/037193
- US-A- 5 608 574
- US-A- 5 608 574

## Beschreibung

### Technisches Gebiet

Die vorliegende Erfindung betrifft einen Montageadapter, nachfolgend als Adapter bezeichnet, zur lösbaren Befestigung einer definierten Aussparung eines Sterilüberzugs an einem Objektiv eines Mikroskops, insbesondere eines Operationsmikroskops, welcher eine schnelle und einfache Montage in nur einer einzigen Montageposition ermöglicht und dennoch eine sichere Befestigung des Sterilüberzugs an dem Mikroskop gewährleistet. Weiterhin betrifft die Erfindung ein System aus einem Operationsmikroskop und einem derartigen Adapter.

### Hintergrund der Erfindung

In der modernen Chirurgie werden zunehmend Operationsmikroskope eingesetzt, welche einem Operateur bzw. Chirurgen eine verbesserte Sicht des Operationsfeldes ermöglichen. Da derartige Mikroskope vor einer Operation jedoch nicht wie andere chirurgische Instrumente sterilisiert werden können, müssen Operationsmikroskope vor einer Operation mit einem Sterilüberzug abgedeckt werden, bzw. in einen Sterilüberzug eingehüllt werden, um Kontaminationen während der Operation aufgrund des selbst nicht sterilen Mikroskops zu verhindern. Derartige Sterilüberzüge sind auch als "Drapes" bekannt. Damit ein derartiger Sterilüberzug zwar ein steriles Arbeiten ermöglicht, aber nicht die Bildqualität negativ beeinflusst, muss der Sterilüberzug im Bereich des Objektivs des Mikroskops zwar geschlossen sein, aber eine lichtdurchlässige Aussparung bzw. ein Fenster (auch als "Drapelinse" bezeichnet) aufweisen, wodurch ein Strahlendurchtritt, insbesondere von Lichtstrahlen, ermöglicht wird. Um das Operationsfeld optimal auszuleuchten, muss das zur Beleuchtung ausgesandte Licht einen ähnlichen Weg nehmen wie das in das Mikroskop entlang der optischen Achse zurückfließende Licht. Hierbei muss das zur Beleuchtung ausgesandte Licht so geleitet werden, dass ungewollte Reflexionen, z.B. ein Spiegelbild der Beleuchtungsquelle, nicht im Bereich des Operationsfelds auftreten.

Eine Möglichkeit, Reflexionen stark zu verringern besteht darin, die lichtdurchlässige Aussparung bzw. das Fenster in dem Sterilüberzug bzw. die "Drapelinse" und/oder umgebende Geometrien mit einer Beschichtung zu versehen, welche Reflexionen reduziert.

Eine weitere Möglichkeit besteht darin, die Drapelinse in einem definierten spitzen Winkel zu der optischen Linse anzuordnen, d.h. in einem Winkel, welcher deutlich von einem rechten Winkel abweicht.

Die Positionierung und Montage der Drapelinse an dem Objektiv des Operationsmikroskops erfolgt mittels eines Adapters, welcher die Drapelinse in einer vorherbestimmten und reproduzierbaren Position relativ zu dem Objektiv des Mikroskops hält bzw. fixiert. Durch diesen Adapter wird somit auch die Winkelposition der Drapelinse zu der optischen Achse bestimmt.

Die Anmelderin der vorliegenden Erfindung hat festgestellt, dass die exakte Positionierung des Adapters an dem Objektiv des Mikroskops demnach die exakte Positionierung der Drapelinse in einer erwünschten Relativpositionierung zu der optischen Achse bedingt, was entscheidend für die Gewährleistung einer gleichbleibend hohen Bildqualität ist. Wird nämlich der Adapter fehlerhaft an dem Objektiv des Mikroskops montiert, beispielsweise verdreht oder verkippt montiert, so können Reflexionen in den Strahlengang geraten, die die Bildqualität beeinträchtigen. Sitzt der Adapter nicht fest genug an dem Objektiv des Mikroskops, so kann sich die Positionierung des Adapters zudem mit der Zeit verändern, was dazu führen kann, dass die Bildqualität während einer Operation abnimmt. Für eine gute Handhabbarkeit und Benutzerfreundlichkeit sollte der Adapter schließlich auch möglichst einfach zu montieren sein.

### Stand der Technik

Verschiedene Adapter zur lösbaren Befestigung einer Drapelinse und eines Drapes an einem Operationsmikroskop sind aus dem Stand der Technik bekannt.

Beispielsweise bedienen sich manche Adapter eines elastischen Formschlusses beispielsweise in Form eines Gummibands oder einer Gummimanschette zwischen dem Adapter und dem Operationsmikroskop, um den Adapter an dem Mikroskop zu befestigen.

Häufig weist hierbei das Mikroskop an dessen Außenfläche Formelemente/Konturen auf, die von entsprechenden Formelementen des Adapters hinterschnitten werden. Beispielsweise kann der Adapter in seinem Innenumfang gedehnt oder verformt werden und in diesem Zustand auf das Mikroskop aufgebracht werden. Danach lässt der Benutzer den Adapter los, welcher sich daraufhin elastisch in Richtung seiner Ausgangskonfiguration zurückverformt und durch die hinterschneidenden Formelemente an dem Mikroskop gehalten wird.

Ein derartiger elastischer Formschluss hat jedoch den Nachteil, dass entweder der Benutzer bei der Montage des Adapters an dem Mikroskop eine hohe Kraft aufbringen muss, z.B. wenn eine starke Verformung des Adapters bzw. der hinterschneidenden Elemente notwendig ist, um den Adapter auf das Mikroskop aufzubringen, oder aber eine erhöhte Wahrscheinlichkeit einer ungewollten Demontage des Adapters von dem Mikroskop besteht, z.B. wenn nur eine geringes Übermaß der hinterschneidenden Elemente vorliegt. Zudem kann der Adapter nicht nur in einer einzigen gewollten und reproduzierbaren Montageposition montiert werden, sondern kann verdreht bzw. verkippt an dem Objektiv angebracht werden.

Ein weiterer aus dem Stand der Technik bekannter Adapter ist im Wesentlichen ein Elastomerelement mit einer eingesetzten Drapelinse. Der Adapter kann nach Art eines breiten Gummirings in Axialrichtung auf das Objektiv eines Mikroskops aufgesetzt werden. Die Montage des Adapters ist einfach und die Anzahl der Komponenten des Adapters ist klein.

Neben den vorstehend geschilderten Problemen eines derartigen elastischen Formschlusses, nämlich einer erhöhten notwendigen Montagekraft bei einem hohen Übermaß und einer erhöhten Wahrscheinlichkeit ungewollter Demontage bei geringem Übermaß, ist auch dieser Adapter nicht nur in einer einzigen gewollten Montageposition montierbar, sondern kann verdreht bzw. verkippt an dem Objektiv angebracht werden.

Die Druckschrift US 5608574 offenbart zudem einen Adapter, der mittels mehrerer Rippen reibschlüssig an dem Objektiv eines Mikroskops gehalten wird. Die Montage des Adapters an dem Objektiv ist einfach, allerdings wird auch bei diesem Adapter keine reproduzierbare einzige Montageposition vorgegeben. Vielmehr kann der auf das Objektiv aufgesteckte Adapter um das Objektiv herum verdreht werden und lediglich zwei korrespondierende Markierungen auf dem Adapter und dem Objektiv dienen dazu, eine erwünschte Montageposition zwar anzuzeigen aber nicht als einzig mögliche Montageposition festzulegen.

Ein weiterer aus dem Stand der Technik bekannter Adapter ermöglicht eine Montage des Adapters auf einem Objektiv eines Mikroskops in lediglich einer einzigen reproduzierbaren Montageposition. Dies wird allerdings durch einen Bajonettverschluss zwischen dem Objektiv und dem Adapter erreicht, was eine erhöhte Anzahl von Elementen voraussetzt und bei der Montage eine genauere bzw. schwierigere Prozedur erfordert, weil eine Drehbewegung statt eine einfache Axialbewegung (zum Aufstecken eines Adapters) erforderlich ist.

Die Druckschrift EP 3 178 438 A1 offenbart einen Schutzglasadapter für ein Operationsmikroskop, das eine erste Anlagefläche aufweist, wobei die erste Anlagefläche derart ausgebildet ist, dass diese mit einer korrespondierenden zweiten Anlagefläche des Operationsmikroskops in Kontakt bringbar ist. Der Schutzglasadapter weist eine Führungsöffnung zur Aufnahme eines Hauptobjektivs des Operationsmikroskops auf, wobei die Führungsöffnung als Kegelstumpf mit einer ersten Zentralachse ausgebildet ist, wobei der größere Durchmesser des Kegelstumpfes zu der ersten Anlagefläche hin ausgebildet ist. Der Schutzglasadapter weist mindestens ein Positionierungselement auf, wobei das mindestens eine Positionierungselement starr ausgebildet ist und an der ersten Anlagefläche oder an der Oberfläche der Führungsöffnung angeordnet ist, wobei das Positionierungselement zur formschlüssigen Aufnahme in einem Gegenelement an dem Operationsmikroskop geeignet ist. Der Schutzglasadapter weist mindestens ein Halteelement auf, wobei durch das Halteelement eine Haltekraft parallel zu der ersten Zentralachse zwischen dem Schutzglasadapter und dem Operationsmikroskop bewirkbar ist, wobei die Haltekraft durch eine Magnetkraft gebildet ist. Die Druckschrift WO 2009/037193 A2 offenbart eine kombinierte Drape/Schutzglas-Anordnung für OP-Mikroskope. Sie besteht aus einer adaptierbaren Schutzglasfassung, welche ein transparentes Schutzglas umfasst, sowie ein mit ihr verbundenes Drape. Das Schutzglas ist in der Schutzglasfassung ortsfest positioniert und das Drape ist mit der Schutzglasfassung fest unter Bildung einer einteiligen Gesamtanordnung verschweißt. Die Schutzglasfassung weist zur justiergenauen Adaption an die Unterseite des OP-Mikroskopgehäuses Ankoppelungsmittel auf, die mit entsprechenden Ausnehmungen im OP-Mikroskop korrespondieren. Zusätzlich sind Fixationshilfen vorgesehen, die passgenau mit entsprechenden Ausnehmungen im Mikroskopgehäuse korrespondieren. Außerdem sind an der Schutzglasfassung seitliche Führungsbolzen als "Andock"-Hilfen vorgesehen.

### Zusammenfassung der Erfindung

Ausgehend von dem vorstehend beschrieben Stand der Technik liegt der vorliegenden Erfindung die Aufgabe zugrunde, einen Adapter zur Befestigung eines Sterilüberzugs mit einer Drapelinse an einem Operationsmikroskop bereitzustellen, welcher mit einer möglichst geringen Anzahl an Komponenten auskommt, dabei jedoch auf einfache Weise und in einer einzigen vorherbestimmten Montageposition reproduzierbar auf einem Objektiv eines Mikroskops montierbar ist und zuverlässig in dieser Montageposition verbleibt.

Diese Aufgabe wird gelöst durch einen Adapter gemäß Anspruch 1, ein Mikroskop nach Anspruch 9 und ein System aus Mikroskop und Adapter gemäß Anspruch 10. Vorteilhafte Weiterbildungen der Erfindung ergeben sich aus den Unteransprüchen.

Der Kerngedanke der vorliegenden Erfindung besteht demzufolge darin, einen (Sterilüberzug-seitigen) Montageadapter (nachfolgend Adapter) zur lösbaren Befestigung eines Sterilüberzugs an einem Objektiv eines Mikroskops bereitzustellen, der mit einer Durchgriffs- oder Durchgangsöffnung (nachfolgend Durchgang) für ein ausschließlich axiales (d.h. kein Drehen erforderlich) Aufstecken auf das Mikroskop bzw. dessen Objektiv ausgebildet ist und der eine in und/oder aus dem Durchgang wahlweise bewegbare oder den Durchmesser/Umfang des Durchgangs wahlweise vergrößernde und/oder verkleinernde Rasteinrichtung hat, die insbesondere dafür vorgesehen und ausgebildet ist, mit einer Geometrie, beispielsweise einem Vor- oder Rücksprung auf Seiten des Mikroskops bzw. dessen Objektivs in hinterschneidenden Rasteingriff zu kommen. Schließlich hat der Adapter zumindest eine Positioniergeometrie, beispielsweise in Form einer Asymmetrie insbesondere in/an dem Durchgang, einer Ausnehmung / eines Rücksprungs oder eines Vorsprungs, die eine (im Wesentlichen exakte) reproduzierbare (automatische/zwangsläufige) Winkelpositionierung oder Rotationsausrichtung des Adapters am Mikroskop / Objektiv ausschließlich bei dessen axialem Aufstecken bewirkt. Dabei wirkt die Positioniergeometrie unabhängig von der Rasteinrichtung und ist von der Rasteinrichtung funktional und/oder örtlich getrennt/separiert. Insofern funktionieren der erfindungsgemäße Adapter und das Mikroskop/Mikroskop-Objektiv nach dem Schlüssel-Schlüsselloch-Prinzip, wonach nur ein Adapter mit einer individuell/gattungsgemäß gestalteten oder bestimmten Positioniergeometrie und ggf. einer vorzugsweise individuell/gattungsgemäß gestalteten oder bestimmten Rasteinrichtung axial (ohne notwendige Drehbewegung) auf ein Mikroskop bestimmter Gattung aufgesteckt wird, und dabei zum Einen den axial sichernden Rasteingriff mit dem Mikroskop/Objektiv herstellt und zum Anderen die relative Winkelposition des Adapters bezüglich des Mikroskops/Objektiv gewährleistet.

Konkreter ausgedrückt bildet die Positioniergeometrie des Adapters bevorzugt wenigstens eine Ausrichtungsaufnahme (z.B. Axialbohrung/Kerbe), in die bei der Montage des Adapters an dem Mikroskop/Objektiv beispielsweise ein entsprechender Eingreifabschnitt, vorzugsweise Ausrichtungsstift bzw. Ausrichtungszapfen oder eine Feder/Leiste am Objektiv des Mikroskops in Axialrichtung eingreift, wenn sich die Ausrichtungsaufnahme und der Eingreifabschnitt (Ausrichtungszapfen, etc.) bei korrekter relativer Winkelposition (Ist-Winkelposition entspricht einer Soll-Winkelposition) von Adapter und Objektiv im Wesentlich axial überlappen/überdecken. Ein erfindungsgemäßes Mikroskop weist demzufolge einen derartigen Eingreifabschnitt vorzugsweise Ausrichtungsstift/Zapfen oder Leiste auf, der dazu ausgelegt ist, bei korrekter relativer Winkelposition von Adapter und Objektiv in die Ausrichtungsaufnahme des erfindungsgemäßen Adapters einzugreifen. Durch diesen Formschluss zwischen der Adapter-seitigen Ausrichtungsaufnahme und dem Mikroskop-seitigen Eingreifabschnitt (Ausrichtungsstift/Zapfen oder Leiste) ist der Adapter nur in vorzugsweise einer einzigen Position (Soll-Winkelposition) an dem Objektiv (durch eine ausschließlich axiale Aufsteckbewegung) montierbar und ist gleichzeitig gegen ein Verdrehen und Verkippen des Adapters relativ zu dem Objektiv gesichert. Letzteres ergibt sich insbesondere aus dem Umstand, dass die Positioniergeometrie (die Ausrichtungsaufnahme) am Adapter radial vom Durchgang beabstandet ist bzw. über den eigentlichen Umfang des Durchgangs radial vorragt, wodurch das Mikroskop/Objektiv bzw. dessen Eingreifabschnitt (Ausrichtungsstift/Zapfen oder Leiste) eine axial wirkende Abstützung auf den Adapter ausüben kann.

Ein erfindungsgemäßer Adapter zur lösbaren Befestigung eines Sterilüberzugs an einem Objektiv eines Mikroskops weist gemäß einem bevorzugten Aspekt der vorliegenden Erfindung eine Grundplatte, welche das bezüglich der Grundplatte gekipptes/angestelltes transparentes Fenster (Drapelinse) enthält oder aufnimmt, eine auf die Grundplatte aufgesetzte Kappe und ein zwischen der Grundplatte und der Kappe angeordnetes bewegliches bzw. verschiebbar aufgenommenes/geführtes Element vorzugsweise in Form einer Zungenplatte auf.

Die Grundplatte, die Kappe und ggf. die Zungenplatte weisen gemäß einem Unteraspekt der vorliegenden Erfindung jeweils eine Durchgriffs-/Durchgangsöffnung auf, die den gemeinsamen Durchgang darstellen, wodurch der Adapter über das Objektiv eines Mikroskops geführt/gesteckt werden kann, wobei durch Verschieben der Zungenplatte relativ zur Grundplatte und Kappe (sowie senkrecht zur Durchgangsöffnung) eine für das axiale Überstülpen/Aufstecken auf das Mikroskop-Objektiv notwendige Deckungsgleichheit der sich überlagernden Durchgangsöffnungen aufgehoben werden kann, um so das Mikroskop-Objektiv in den nunmehr relativ zueinander verschobenen Durchgangsöffnungen zu verspannen. In anderen Worten ausgedrückt kann der Adapter aus zwei (flächig) aneinander befestigten Platten-/Rahmenelementen (Grundplatte und Kappe) bestehen, die jeweils eine Durchgangsöffnung vorzugsweise mit zueinander im Wesentlich gleichem Durchmesser aufweisen, wobei zwischen den Platten-/Rahmenelementen ein Spalt/Fach erhalten bleibt, in welchem die Zungenplatte (Rastzunge/Riegel) verschiebbar aufgenommen ist, mittels welcher die Durchgangsöffnungen im Spaltbereich eingeengt werden können. Die Zungenplatte (Rastzunge/Riegel) bildet somit die erfindungsgemäße Rasteinrichtung. Sie kann als einfacher Riegel oder als Platte ebenfalls mit Durchgangsöffnung ausgeformt sein.

Zur Sicherstellung einer korrekten Montage des Adapters am Objektiv eines Mikroskops weist der Adapter erfindungsgemäß die Ausrichtungsaufnahme bzw. Positionierungsaufnahme vorzugsweise in Form einer Bohrung oder einer randseitigen Kerbe auf, welche dazu ausgelegt ist, einen entsprechenden Eingreifabschnitt (Ausrichtungszapfen / -stift bzw. Positionierungszapfen / -stift) des Mikroskop-Objektivs aufzunehmen. Durch den Formschluss zwischen der Ausrichtungsaufnahme des Adapters und dem Eingreifabschnitt (Ausrichtungszapfen) des Mikroskops wird sichergestellt, dass der Adapter in vorzugsweise nur einer einzigen möglichen Montageposition an dem Objektiv montier werden kann und bei einer wiederholten Montage des Adapters an dem Objektiv stets die gleich Montageposition des Adapters an dem Mikroskop erreicht wird. Die Ausrichtungsaufnahme dient somit als Verdrehsicherung und Verkippsicherung bei der Montage des Adapters an dem Mikroskop.

Es kann bei dem erfindungsgemäßen Adapter nur eine einzige Ausrichtungsaufnahme vorgesehen sein, oder es können auch mehrere Ausrichtungsaufnahmen vorgesehen sein, die jeweils mit einem entsprechenden Eingreifabschnitt wie Ausrichtungszapfen oder dergleichen Geometrie am Objektiv zusammenwirken. Bei mehreren Ausrichtungsaufnahmen können diese alle gleich sein, sich aber alternativ dazu auch in ihrer Ausgestaltung, wie z.B. Tiefe, Geometrie, Flächenmaßen, voneinander unterscheiden. Dadurch ist es möglich, den Adapter für Mikroskope unterschiedlicher Gattung mit ggf. unterschiedlich zueinander angeordneten/ausgebildeten Eingreifabschnitten universell verwendbar zu machen.

Die Grundplatte hat bevorzugt einen röhrenförmigen / zylindrischen Abschnitt oder Stutzen, welcher an seiner einen axialen Stirnseite durch ein optisch transparentes Element (Fenster) geschlossen/verschließbar ist und an seiner dem optisch transparenten Element abgewandten Stirnseite eine Öffnung mit einem umlaufenden Flansch oder Kragen aufweist, der die Grundplatte letztlich bildet. Der zylindrische Abschnitt dient bei der Benutzung des Adapters bevorzugt zur Aufnahme des Objektivs eines Mikroskops, wenn der Adapter auf bzw. radial außenseitig über das Objektiv axial aufgesteckt wird. Das optisch transparente Element ist das eigentliche Fenster bzw. die Drapelinse, durch welche(s) die Beleuchtung des Operationsfelds und die Betrachtung des Operationsfelds erfolgt. Vorzugsweise verläuft eine Ebene des optisch transparenten Elements in einem spitzen Winkel, wie z.B. 15-25°, vorzugsweise 20°, zu einer Ebene der Grundplatte bzw. insbesondere des Flansches/Kragens, der die Grundplatte bildet.

Die Kappe ist bevorzugt an der Grundplatte angebracht bzw. auf diese aufgesetzt / aufgesteckt /befestigt /verrastet und hat die vorstehend genannte Durchgangsöffnung, welche mit der Durchgangsöffnung des zylindrischen / rohrförmigen Abschnitts / Körpers der Grundplatte fluchtet und somit mit dieser einen gemeinsamen Durchgang bildet. Die Kappe ist bevorzugt in einer festen Relativpositionierung zu der Grundplatte angeordnet bzw. fixiert. Die Kappe kann auch einfach eine vorzugsweise parallel zu der Grundplatte angeordnete Platte sein.

An dieser Stelle sein darauf hingewiesen, dass die Drapelinse quasi einstückig mit der Grundplatte vorgesehen sein kann, beispielsweise indem die Drapelinse in /an den Stutzen der Grundplatte endseitig angeklebt/angeschweißt wird. Es ist aber gemäß einem ggf. unabhängig zu beanspruchenden Aspekt der vorliegenden Erfindung auch möglich, die Drapelinse im Stutzen zu verklemmen/verspannen. Letzteres lässt sich auf einfache Weise dadurch verwirklichen, indem die Grundplatte zweiteilig gestaltet ist, wobei der Stutzen der Grundplatte innenseitig einen Vorsprung ausbildet, der bereits einen entsprechenden Anstell-/Neigungswinkel zur Stutzenlängsachse definiert und als Auflager für die Drapelinse dient. Ferner kann der zweite Teil der Grundplatte, welcher mit dem Stutzen ggf. verrastet wird, einen Andrückabschnitt beispielsweise in Form von Andrückfüßen oder einem Andrückring aufweisen, die/der bei einem Zusammenbau der Grundplatte gegen die Drapelinse anliegen und dabei die bereits lose eingelegte Drapelinse zwischen sich und dem Auflager einspannen. Alternativ hierzu ist es aber auch möglich, die Kappe mit einem entsprechenden Andrückabschnitt z.B. in Form von Andrückfüßen oder einem Ring/Leiste (Stoffeinstückig) auszubilden, die/der bei einem Zusammenbau von Grundplattte und Kappe an der Drapelinse anliegen und so die Drapelinse gegen das Auflager drücken.

Im Konkreten, kann die Drapelinse mit wenigstens einem radialen Vorsprung oder einer radial sich erstreckenden Lasche ausgebildet sein, die in einen im Bereich des Stutzen-seitigen Vorsprungs ausgebildeten Ausbruch an der Stutzenwand eingreift. Auf dem zur Lasche diametral gegenüberliegenden Umfangsabschnitt wirkt dann der Andrückabschnitt an der Kappe ein. Da die Drapelinse bezüglich der Stutzenachse in einem schrägen Winkel eingebaut ist, kann die Stutzenlänge so gewählt sein, dass die eingelegte Drapelinse an ihrem der Lasche diametral gegenüberliegenden Umfangsabschnitt die eine (obere) Stirnseite des Stutzens im Bereich des diesen umgebenden Kragens (im Wesentlichen) erreicht. Auf diese Weise muss der in diesem Umfangsabschnitt ausgebildete Andrückabschnitt an der Kappe nicht in den Stutzen hineinragen, um die Drapelinse zwischen sich und der Grundplatte zu verspannen. Vorzugsweise sind zwei diametral angeordnete, radial vorragende Laschen angeordnet, von denen der eine in den seitlichen Stutzenausbruch unmittelbar oberhalb des stutzen-seitigen Innenvorsprungs eingreift, wobei auf den anderen die Kappe klemmend einwirkt.

Zwischen der Grundplatte und der Kappe bildet sich, wie vorstehend bereits ausgeführt wurde, gemäß einem Unteraspekt der vorliegenden Erfindung eine Art Einschubschacht aus, in den das bewegliche Element/Plattenriegel bzw. die Zungenplatte (planparallel zur Kappe und/oder der Grundplatte) eingeschoben ist, welches bevorzugt ebenfalls eine Durchgangsöffnung hat, welche mit der Durchgangsöffnung der Kappe und der Durchgangsöffnung des zylindrischen Körpers/Stutzens der Grundplatte achsparallel ausrichtbar ist. In anderen Worten kann das bewegliche Element vorzugsweise so zu der Kappe und der Grundplatte ausgerichtet / verschoben werden bzw. sein, dass die Öffnung des beweglichen Elements, die Öffnung der Kappe und die Öffnung des zylindrischen Körpers deckungsgleich sind und zusammen einen gemeinsamen Durchgang bilden.

Das bewegliche Element kann relativ zu der Grundplatte und der Kappe derart bewegbar sein, dass es reversibel aus einer vorherbestimmten Offenstellung, in welcher das bewegliche Element den Durchmesser des Durchgangs nicht beeinflusst (also Deckungsgleich mit den anderen Öffnungen ist), vorzugsweise durch eine Federkraft in eine vorherbestimmte Arretierungsstellung bringbar ist, in welcher das bewegliche Element den Durchmesser des Durchgangs auf vorherbestimmte Weise verringert (achsparallel verschoben ist). Beispielsweise kann das bewegliche Element zwischen der Grundplatte und der Kappe in dem dazwischen ausgebildeten Schacht vorzugsweise federvorgespannt so verschoben werden, dass es teilweise in den Durchgang hineinragt und so den Durchmesser des Durchgangs auf vorherbestimmte Weise verringert.

Vorzugsweise weist der Adapter demzufolge auch mindestens ein Vorspannelement, beispielsweise in Form einer Schraubenfeder oder Druckfeder, auf, welches den Adapter in die Arretierungsstellung vorspannt, d.h. die Zungenplatte relativ zu der Grundplatte und der Kappe in eine Position vorspannt, in der die Durchgangsöffnungen nicht mehr deckungsgleich sind.

Um den Adapter auf das Objektiv eines Mikroskops aufzustecken bzw. aufzusetzen, kann ein Benutzer beispielswiese durch Aufbringen einer Druck-/Schiebekraft auf das bewegliche Element/Zungenplatte, das bewegliche Element entgegen der Vorspannkraft der Vorspannelemente aus der Arretierungsstellung (nicht deckungsgleich angeordnete Durchgangsöffnungen) in die Offenstellung (deckungsgleich angeordnete Durchgangsöffnungen) überführen. In der Offenstellung ist gemäß einem bevorzugten Aspekt der Erfindung der gemeinsame Durchgang demzufolge maximal geöffnet und der Adapter kann durch den Benutzer auf einfache Weise und ohne viel Kraft aufzubringen in axialer Richtung des Objektivs eines Mikroskops auf das Objektiv aufgesetzt / aufgesteckt / übergeschoben werden. Dabei greift der am Objektiv vorgesehene Zapfen oder Vorsprung in die Ausrichtungsaufnahme, vorzugsweise gebildet durch eine Kerbe oder Bohrung an der Grundplatte und/oder an der Kappe formschlüssig ein und stellt so eine vordefinierte Rotationswinkel-Relativlage (Stellung/Montageposition) zwischen Objektiv und Adapter sicher.

Sitzt der Adapter in der erwünschten Stellung / Montageposition auf dem Objektiv, so hört der Benutzer auf, die Schiebekraft auf das bewegliche Element entgegen der Federvorspannung auszuüben und das mindestens eine Vorspannelement führt das bewegliche Element zurück in die Arretierungsstellung, sodass der Adapter an dem Objektiv, bei erneuter Betätigung wieder lösbar aber in unbetätigtem Zustand axialfest gehalten wird.

Der Aufbau des erfindungsgemäßen Adapters erlaubt somit eine einfache und schnelle Montage, da der Adapter nicht durch eine Drehbewegung des Benutzers rotatorisch an dem Objektiv befestigt werden muss, sondern die Montage in axialer Richtung, vorzugsweise durch ein einfaches Aufstecken auf das Objektiv des Mikroskops, erfolgt.

Vorzugsweise erfolgt die vorübergehende Fixierung des Adapters an dem Objektiv durch die Verringerung des Durchmessers des Durchgangs in dem Adapter, vorzugsweise durch eine Relativbewegung des beweglichen Elements zu der Grundplatte und / oder der Kappe. Durch die Verringerung des Durchmessers kann beispielsweise das Objektiv in dem Adapter durch Reibschluss festgeklemmt werden. Alternativ kann aber auch das Objektiv eine Aussparung aufweisen, beispielsweise in Form einer umlaufenden ringförmigen Rille / Nut, in welche das bewegliche Element in der Arretierungsposition eingreift, wodurch der Adapter durch Formschluss an dem Objektiv gehalten wird.

An dieser Stelle sei darauf hingewiesen, dass die Anordnung der vorstehend beschriebenen Zungenplatte nur eine Möglichkeit der wahlweisen Verringerung des Durchgangs-Durchmessers ist. Alternativ könnte auch eine Art (Fächer-)Rosette oder auch ein einfacher elastischer Dichtring angeordnet sein. Auch die Anordnung einer vorzugsweise manuell aufweitbaren Spiral- oder Schenkelfeder wäre denkbar.

Gemäß einem bevorzugten Aspekt der Erfindung sind die Durchgangsöffnung des zylindrischen Körpers/Stutzens der Grundplatte und die Durchgangsöffnung der Kappe kreisförmig ausgebildet und haben im Wesentlichen den gleichen Durchmesser. Die Durchgangsöffnung des zylindrischen Körpers der Grundplatte und die Durchgangsöffnung der Kappe bilden somit einen gemeinsamen Durchmesser von im Wesentlichen einheitlichem Durchmesser. Vorzugsweise hat die Öffnung des beweglichen Elements einen größeren Durchmesser als die Öffnungen des zylindrischen Körpers und der Kappe. Die Öffnung des beweglichen Elements kann aber auch den gleichen Durchmesser wie die Öffnungen des zylindrischen Körpers und der Kappe haben.

Die Öffnung des beweglichen Elements kann kreisförmig sein. Ist die Öffnung des beweglichen Elements nicht kreisförmig, so weist sie bevorzugt doch zumindest abschnittsweise eine gerundete bzw. gekrümmte Außenkontur auf, vorzugsweise die jedoch weniger gerundet bzw. gekrümmt ist als die jeweiligen kreisförmigen Öffnungen des zylindrischen Körpers und der Kappe, damit eine größere Wirkfläche mit dem Objektiv bereitgestellt werden kann, als dies beispielsweise bei einer geraden Kontur möglich wäre.

Um einem Benutzer die Bedienung bzw. Montage des Adapters möglichst zu erleichtern, kann das bewegliche Element einen Betätigungsabschnitt aufweisen, mittels dem ein Benutzer, vorzugsweise durch das Ausüben von Druck, das bewegliche Element aus der vorherbestimmten Arretierungsstellung in die vorherbestimmte Offenstellung bringen kann. Der Betätigungsabschnitt kann beispielsweise ergonomisch an den Finger oder die Hand des Benutzers angepasst sein und vorzugsweise als Fingermulde ausgestaltet sein, an welche der Benutzer seinen Daumen zum Aufbringen von Druck anlegen kann.

Damit der Betätigungsabschnitt und somit der Daumen des Benutzers bei einer Positionierung bzw. Montage des Adapters an dem Mikroskop nicht hinderlich ist, ist der Betätigungsabschnitt gemäß einem Aspekt der Erfindung auf der der Ausrichtungsaufnahme gegenüberliegenden Seite des Adapters über den durch die Öffnungen des zylindrischen Körpers, der Kappe und des beweglichen Elements gebildeten Durchgang hinweg angeordnet. Die Anordnung der Ausrichtungsaufnahme und des Betätigungsabschnitts relativ zueinander dient somit dazu, den Adapter ergonomisch an eine Handhabung durch den Benutzer anzupassen und die Montage des Adapters so einfach wie möglich zu gestalten.

Gemäß einem weiteren Aspekt der Erfindung bilden die Grundplatte und die Kappe gemeinsam die Ausrichtungsaufnahme (Einschubschacht) aus. In anderen Worten bilden die Grundplatte und die Kappe jeweils einen Teil / Teilbereich bzw. Abschnitt der Ausrichtungsaufnahme aus.

Vorzugsweise erstreckt sich hierbei die Ausrichtungsaufnahme durch die Kappe bis hin zu der der Kappe zugewandten Oberfläche des Flansches / der Grundplatte. In anderen Worten erstreckt sich die Ausrichtungsaufnahme in den durch die Grundplatte und die Kappe gebildeten Körper des Adapters hinein und ist nicht lediglich eine oberflächliche Delle oder Mulde in der Oberfläche der Kappe. Dadurch wird ein sicheres Eingreifen des Eingreifabschnitts (Ausrichtungszapfens bzw. Ausrichtungsstifts) in die Ausrichtungsaufnahme gewährleistet.

Um zu verhindern, dass das bewegliche Element aus der Offenstellung in eine Position überführt wird, welche den Durchmesser des Durchgangs zu sehr verringert, d.h. in eine falsche Arretierungsstellung, kann die Grundplatte weiterhin mindestens ein Begrenzungselement bzw. einen Anschlag vorzugsweise in Form eines Vorsprungs aufweisen, welches /welcher die Bewegung des beweglichen Elements relativ zu dem zylindrischen Körper und der Kappe aus der vorherbestimmten Offenstellung in die vorherbestimmte Arretierungsstellung begrenzt. Dadurch wird auch verhindert, dass das bewegliche Element (Zungenplatte) aus dem Einschubschacht unbeabsichtigt herausfällt.

In anderen Worten kann das bewegliche Element nur so weit aus der Offenstellung wegbewegt werden, bis es in Kontakt mit dem Begrenzungselement bzw. Anschlag kommt und die korrekte Arretierungsstellung erreicht ist. Das bewegliche Element kann in anderen Worten beispielsweise nur soweit aus der Offenstellung in Richtung hin zu dem Durchgang verschoben werden, bis es an dem Begrenzungselement bzw. Anschlag anstößt und somit in der korrekten Arretierungsstellung gestoppt wird.

Ein weiterer ggf. unabhängig zu beanspruchender Aspekt der Erfindung betrifft ein (medizinisches) Mikroskop, welches dazu ausgelegt ist, mit einem erfindungsgemäßen Adapter verwendet zu werden. Ein derartiges Mikroskop weist an seinem Objektiv mindestens einen Eingreifabschnitt vorzugsweise in Form eines Ausrichtungszapfens bzw. Ausrichtungsstifts oder einer Leiste auf oder ist mit einem solchen bestückbar/nachrüstbar, welcher dazu ausgelegt ist, in die Ausrichtungsaufnahme des Adapters aufgenommen bzw. mit dieser in Eingriff gebracht zu werden, wodurch eine bestimmte eindeutige Montageposition des Adapters an dem Mikroskop festgelegt wird. Das Mikroskop kann bevorzugt an seinem Objektiv mehrere Eingreifabschnitte/ Ausrichtungszapfen bzw. Ausrichtungsstifte aufweisen.

Der Eingreifabschnitt/Ausrichtungszapfen ist vorzugsweise in seinen Abmessungen derart ausgelegt, dass er (möglichst) spielfrei in die Ausrichtungsaufnahme des Adapters passt. Der Eingreifabschnitt/Ausrichtungszapfen ist dazu ausgestaltet, mit der Ausrichtungsaufnahme einen Formschluss zu bilden, oder auch mit der Ausrichtungsaufnahme in, gegebenenfalls hinterschneidenden, Eingriff zu treten. Der Eingreifabschnitt/Ausrichtungszapfen dient somit als Sicherung gegen ein Verdrehen und / oder Verkippen des Adapters auf dem Objektiv.

Ein weiterer Aspekt der Erfindung betrifft ein System aus einem erfindungsgemäßen Adapter und einem erfindungsgemäßen Mikroskop, wie sie vorstehend beschrieben sind.

### Figurenbeschreibung

Nachfolgend werden bevorzugte Ausführungsformen eines erfindungsgemäßen Adapters mit Bezug auf die Figuren 1a bis 5b und 6 beschrieben. Hierbei zeigt / zeigen
Figur 1a einen erfindungsgemäßen Adapter in der Offenstellung;
Figur 1b einen erfindungsgemäßen Adapter in der Arretierungsstellung;
die Figuren 2a bis 2c verschiedene Ansichten eines erfindungsgemäßen Adapters in der Offenstellung;
die Figuren 3a bis 3b eine Grundplatte eines erfindungsgemäßen Adapters;
die Figuren 4a und 4b eine Kappe eines erfindungsgemäßen Adapters;
die Figuren 5a bis 5b ein bewegliches Element eines erfindungsgemäßen Adapters,
Fig. 6 ein (medizinisches) Mikroskop mit Objektiv, an dem ein Positionierzapfen/Vorsprung angebracht oder ausgebildet ist,
Fig. 7 eine Grundplatte für ein loses Einlegen einer Drapelinse und anschließendes Verspannen mittels einer Kappe und
Fig. 8 eine Drapelinse für ein loses Einlegen in die Grundplatte nach Fig. 7.

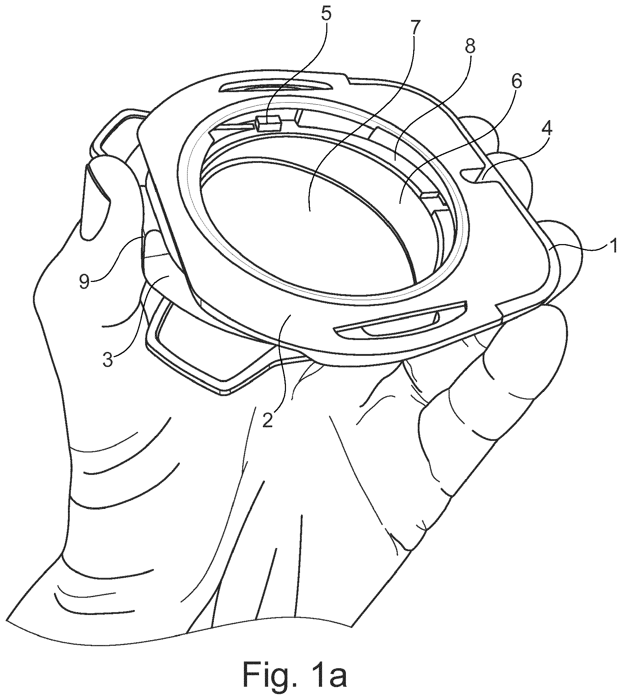

Wie in Figur 1a gezeigt, weist ein erfindungsgemäßer Adapter eine Grundplatte 1, eine (flächig) darauf aufgesetzte Kappe 2 und ein zwischen der Grundplatte 1 und der Kappe 2 angeordnetes bewegliches Element/Zungenplatte 3 auf.

Die Grundplatte 1 weist einen hohlzylindrischen Körper oder Stutzen 6 auf, welcher an seiner einen (unteren) Stirnseite durch ein optisch transparentes Element 7 geschlossen ist und an seiner dem optisch transparenten Element 7 abgewandten (oberen) Stirnseite eine Öffnung 10 mit einem umlaufenden Flansch oder Kragen 8 aufweist, der die Grundplatte 1 bildet, wobei vorzugsweise eine Ebene des optisch transparenten Elements 7 in einem (spitzen) Winkel von 15°-25°, bevorzugt ca. 20°, zu einer (senkrecht zur Stutzenachse ausgerichteten) Ebene des Flansches 8 verläuft.

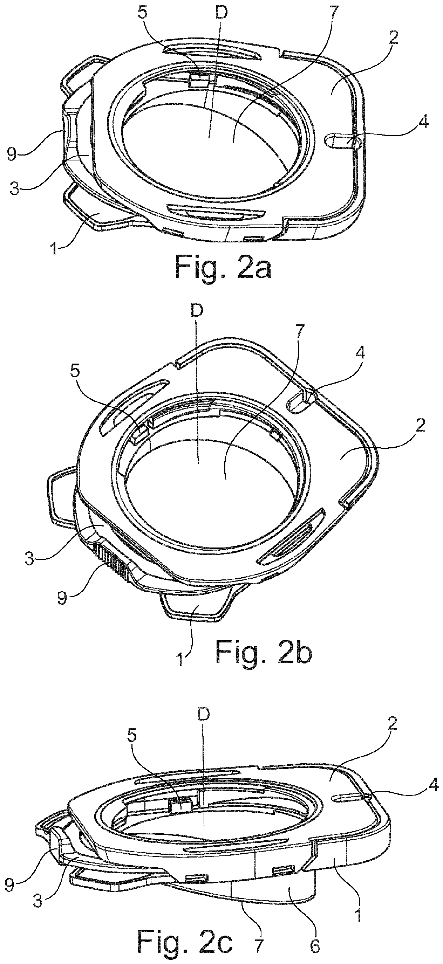

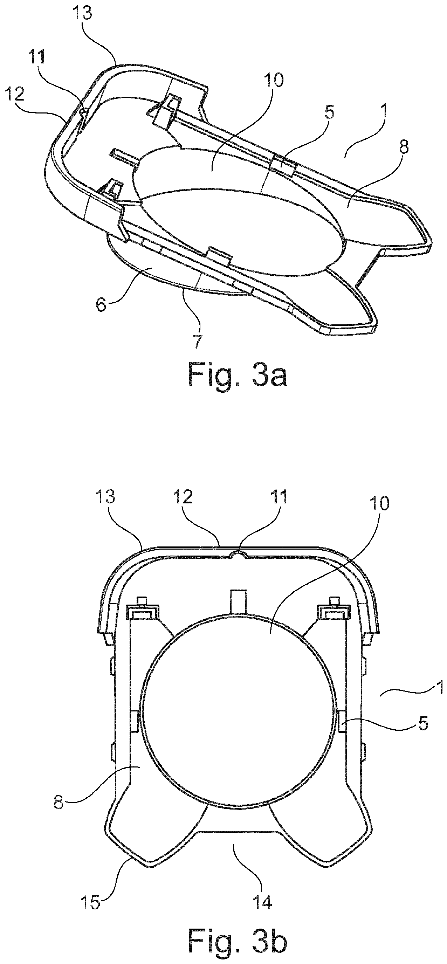

Wie besonders in den Figuren 2a bis 2c und 3a bis 3b erkennbar, weisen die Grundplatte 1 und die Kappe 2 jeweils eine (Durchgangs-)Öffnung 10 bzw. 17 auf, welche coaxial zueinander ausgerichtet sind und zusammen einen gemeinsamen Durchgang D ausbilden. Der gemeinsame Durchgang D kann quasi eine Verlängerung des zylindrischen Körpers 6 der Grundplatte 1 über den umlaufenden Flansch 8 hinweg sein. Der gemeinsame Durchgang D erstreckt sich somit durch die Öffnungen 10 und 17 der Grundplatte 1 und der Kappe 2 hin zu dem optisch transparenten Element 7.

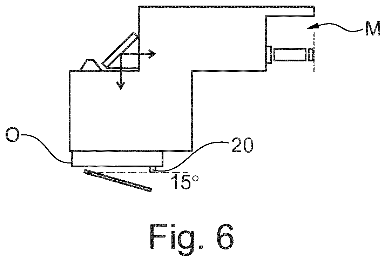

Das bewegliche Element 3 weist ebenfalls eine Öffnung 18 auf, welche bezüglich der Öffnungen 10 bzw. 17 des zylindrischen Körpers 7 der Grundplatte 1 und der Kappe 2 ausgerichtet werden kann, sodass die/alle Öffnungen 10, 17, 18 des zylindrischen Körpers der Grundplatte 1, der Kappe 2 und des beweglichen Elements 3 miteinander fluchten und den gemeinsamen Durchgang D bilden. In diesen gemeinsamen Durchgang D wird das Objektiv O eines Mikroskops M (siehe Fig. 6) eingeführt, wenn der Adapter auf das Objektiv O des Mikroskops M in axialer Richtung aufgesetzt bzw. aufgesteckt wird. In der Offenstellung des Adapters ist der gemeinsame Durchgang daher so weit geöffnet bzw. hat einen derartigen Durchmesser, dass das Objektiv O des Mikroskops M in den Adapter eingeführt werden kann.

In der Figur 1a ist zudem eine Positioniergeometrie in Form einer Ausrichtungsaufnahme 4 gezeigt, die vorzugsweise von der Kappe 2 und der Grundplatte 1 gemeinsam ausgebildet wird. Diese Ausrichtaufnahme 4 ist im Radialabstand zum Durchgang D in zumindest einem Umfangsabschnitt bezüglich des Durchgangs angeordnet.

In dem der Ausrichtungsaufnahme 4 diametral gegenüberliegenden Umfangsabschnittsbereich des Adapters steht das bewegliche Element 3 über den Außenrand der Kappe 2 und der Grundplatte 1 hervor und weist in dem vorragenden Bereich einen Betätigungsabschnitt 9 auf, welcher im vorliegenden Fall eine ergonomische Mulde ist, in welcher ein Daumen eines Benutzers platziert werden kann, wie dies in Figur 1a gezeigt ist.

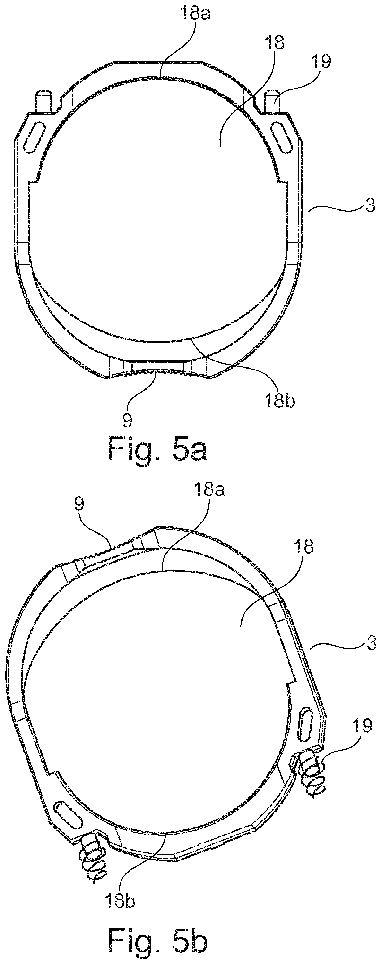

In der in Figur 1a gezeigten Darstellung übt der Benutzer mit seinem Daumen Druck auf den Betätigungsabschnitt 9 des beweglichen Elements 3 aus. Der Adapter wird durch das Ausüben von Druck auf den Betätigungsabschnitt 9 (dadurch wird die Zungenplatte entgegen der Vorspannung von Federn (siehe Fig. 5b) in einen Aufnahmeschacht zwischen Grundplatte 1 und Kappe 2 hineingedrückt) in eine Offenstellung gebracht, in welcher das bewegliche Element den Durchgang D nicht einengt.

In der in der Figur 1a gezeigten Ansicht befindet sich der Adapter in der Offenstellung, in welcher der Durchmesser des gemeinsamen Durchgangs D im Wesentlichen nur durch die Abmessung der Öffnung 10 des zylindrischen Körpers 6 der Grundplatte 1 und der Öffnung 17 der Kappe 2 begrenzt wird. In der Offenstellung hat das bewegliche Element 3 somit keinen Einfluss auf den Durchmesser des gemeinsamen Durchgangs D.

In der vorliegenden Ausführungsform sind die Öffnungen 10 und 17 kreisförmig und haben im Wesentlichen den gleichen Durchmesser. Zudem sind die Grundplatte 1 und die Kappe 2 fest miteinander verbunden, beispielsweise durch randseite Rastnasen an der Grundplatte 1, die in randseitige Hinterschneidungen an der Kappe 3 eingreifen/einclicken, sodass auch die Relativpositionierung der Öffnungen 10, 17 zueinander fest vorgegeben bzw. unveränderlich ist.

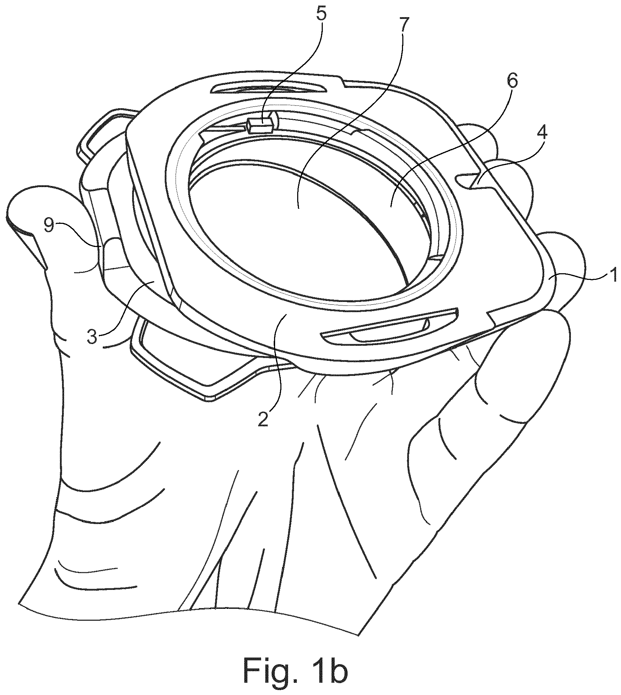

In der Figur 1a sind zudem die Begrenzungselemente (Anschläge) 5 der Grundplatte 1 gezeigt, welche eine Bewegung des beweglichen Elements 3 aus der in der Figur 1a gezeigten Offenstellung in eine in Figur 1b gezeigte Arretierungsstellung begrenzen und so auch ein Herausfallen des beweglichen Elements 3 aus dem Einschubschacht zwischen der Grundplatte 1 und der Kappe 2 verhindern. In anderen Worten begrenzen die Begrenzungselemente 5 eine Bewegung des beweglichen Elements 3, welche den Durchmesser des gemeinsamen Durchgangs D verringert bzw. den Durchgang D verengt. In wieder anderen Worten begrenzen die Begrenzungselemente 5 eine Bewegung des beweglichen Elements 3 radial in den Durchgang D hinein. Alternativ oder zusätzlich können auch Begrenzungselemente an der Kappe 2 vorgesehen sein (nicht weiter dargestellt).

Wie in Figur 1a ersichtlich, sind diese Begrenzungselemente 5 jeweils als ein Vorsprung bzw. als jeweils ein Anschlag ausgebildet. In den in Figur 1a und 1b gezeigten Ansichten ist nur ein einziges derartiges Begrenzungselement 5 zu sehen. Tatsächlich kann bei einer Ausführungsform der vorliegenden Erfindung nur ein einziges derartiges Begrenzungselement 5 vorgesehen sein. Alternativ können jedoch auch mehrere derartige Begrenzungselemente 5 bzw. Anschläge vorgesehen sein. Die Begrenzungselemente 5 können gleichartig oder voneinander unterschiedlich sein.

Wie in der Figur 1a gezeigt, weist der erfindungsgemäßer Adapter die vorstehend genannte Ausrichtungsaufnahme 4 auf. Bei dem erfindungsgemäßen Adapter kann lediglich eine einzige Ausrichtungsaufnahme 4 vorgesehen sein, es können jedoch auch mehrere gleichartige oder voneinander unterschiedlich geformte/dimensionierte Ausrichtungsaufnahmen 4 in unterschiedlichen Umfangsabschnitten bezüglich des Durchgangs 4 vorgesehen sein.

Die Figur 1b zeigt den erfindungsgemäßen Adapter in einer Arretierungsstellung. Wie in dieser Figur gezeigt, ragt in der Arretierungsstellung das bewegliche Element 3 zumindest abschnittsweise in den durch die Öffnungen 10, 17 des zylindrischen Körpers der Grundplatte 1 und der Kappe 2 gebildeten Durchgang D radial hinein und engt so die Öffnung dieses Durchgangs D ein. Wird der Adapter an einem Objektiv O eines Mikroskops M montiert, so hinterschneidet das in den Durchgang D hereinragende bewegliche Element 3 beispielsweise einen nicht weiter gezeigten Ringvorsprung des Objektivs O, wodurch der Adapter an dem Objektiv axial gehalten wird.

Wie in der Figur 1b ebenfalls klar ersichtlich, liegt in der Arretierungsstellung das bewegliche Element 3 vorzugsweise an dem Begrenzungselement 5 an, das heißt, es befindet sich in Kontakt mit dem Begrenzungselement 5, welches daher eine Bewegung des beweglichen Elements 3 in der Richtung aus der Offenstellung in die Arretierungsstellung, welche in der Figur 1b der Richtung nach links entspricht, begrenzt.

Wie zudem in der Figur 1b gezeigt ist, muss ein Benutzer in der Arretierungsstellung mit dem Daumen keinen Druck auf den Betätigungsabschnitt 9 des beweglichen Elements 3 des Adapters ausüben. Wenn kein Druck auf den Betätigungsabschnitt 9 des beweglichen Elements 3 aufgebracht wird, überführen die Vorspannelemente bzw. Rückführelemente, welche beispielsweise als Schraubenfeder oder Druckfeder gemäß der Fig. 5b ausgestaltet sind, und zwischen dem beweglichen Element 3 und der Grundplatte 1 und / oder der Kappe 2 angeordnet sind, das bewegliche Element 3 selbsttätig aus der Offenstellung in die Arretierungsstellung.

Für die Montage des Adapters an dem Objektiv des Mikroskops bedeutet das, dass der Adapter, während Druck auf den Betätigungsabschnitt 9 des beweglichen Elements 3 aufgebracht wird (d.h. der Adapter sich in Offenstellung befindet), auf das Objektiv entlang der axialen Richtung des Objektivs O aufgesteckt bzw. aufgebracht wird.

Ist der Adapter an dem Objektiv O korrekt poitioniert, so wird kein Druck mehr auf den Betätigungsabschnitt 9 des beweglichen Elements 3 ausgeübt (der Benutzer hört auf, auf den Betätigungsabschnitt 9 des beweglichen Elements zu drücken). Die Vorspannelemente bzw. Rückführelemente überführen daraufhin den Adapter in die Arretierungsstellung, indem sie das bewegliche Element 3 derart bewegen, dass der Durchmesser des Durchgangs D durch das in diesen hereinragende bewegliche Element 3 verringert wird, wodurch der Adapter sicher an dem Objektiv des Mikroskops verankert wird. In der Arretierungsstellung ist der Adapter somit lösbar aber axial fest an dem Objektiv O des Mikroskops M montiert.

In den Figuren 2a bis 2c sind verschiedene Ansichten eines erfindungsgemäßen Adapters in Offenstellung gezeigt.

In den Figuren 2a bis 2c ist gezeigt, dass die (langlochartige) Ausrichtungsaufnahme 4 co-axial zum Durchgang D durch die Kappe 2 und die Grundplatte 1 gemeinsam, das heißt gemeinschaftlich bzw. zusammenwirkend, ausgebildet wird. Alternativ wäre es jedoch auch möglich, die Ausrichtungsaufnahme nur durch die Kappe 2 oder die Grundplatte 1 auszubilden.

Bevorzugt ist es weiterhin, dass sich die Ausrichtungsaufnahme 4 weit genug in den Körper des Adapters achsparallel zur Durchgangsöffnung D hineinerstreckt, um eine sichere Aufnahme eines entsprechend geformten Eingreifabschnitts, wie z.B. Ausrichtungszapfens bzw. Ausrichtungsstifts A des Objektivs O des Mikroskops M zu gewährleisten. In der gezeigten Ausführungsform verläuft die Ausrichtungsaufnahme 4 beispielsweise von der oberen Oberfläche der Kappe 2 bis zu der der Kappe 2 zugewandten Oberfläche des umlaufenden Flansches 8 der Grundplatte 1. Durch diese Tiefe wird gewährleistet, dass der Ausrichtungszapfen A sicher in die Ausrichtungsaufnahme 4 eingreifen kann und beispielsweise nicht unkontrolliert aus der Ausrichtungsaufnahme 4 herausgleiten kann. Vorzugsweise ist der Greifabschnitt so ausgebildet und/oder angeordnet, insbesondere der Ausrichtungszapfen so lang ausgebildet, dass er bei falscher Orientierung des Adapters diesen derart weit auf axiale Distanz zur Rastgeometrie am Objektiv hält, dass ein Arretieren/Verrasten des beweglichen Körpers am Objektiv nicht möglich oder zumindest deutlich erschwert ist. Somit kann der Adapter nur in einer (einzigen) vorherbestimmten Montageposition an dem Objektiv fixiert bzw. gehalten werden.

An dieser Stelle sei darauf hingewiesen, dass der Adapter sowie der Greifabschnitt/ Ausrichtungszapfen separate sowie getrennt voneinander verkäufliche Bauteile sein können, die in diesem Fall gemeinsam/in deren Zusammenwirken ein Positioniersystem bilden. Es ist aber auch möglich, entsprechende, bereits vorhandene Geometrien an einem Mikroskop/Objektiv als Greifabschnitt zu nutzen und die Positioniergeometrie/Ausrichtungsaufnahme entsprechend zu formen und zu platzieren.

Zusätzlich kann, wie beispielsweise in der Figur 2b gezeigt, der Betätigungsabschnitt 9 des beweglichen Elements 3 für die Handhabung durch den Benutzer weiter optimiert sein, beispielsweise durch eine Veränderung der Oberfläche des Betätigungsabschnitts 9 zur Gewährleistung einer verbesserten Haptik oder durch die Anbringung einer ähnlichen Geometrie auf dem zylindrischen Abschnitt des Bauteils 2 oder einer ähnlichen Fläche, die die Auflage für die anderen Finger bilden.

Wie in Figur 2b gezeigt, können für einen sicheren Griff beispielsweise Rillen, Nuten und / oder bevorzugt rippenartige Vorsprünge an dem Betätigungsabschnitt 9 vorgesehen sein.

Die Figuren 3a und 3b zeigen eine Grundplatte 1 eines erfindungsgemäßen Adapters in detaillierter Form.

Wie vorstehend beschrieben, weist die Grundplatte 1 den zylindrischen Körper/Stutzen 6 auf, der an seinem einen (unteren) Ende das optisch transparente Element (Drapelinse) 7 aufweist, d.h. die Drapelinse bzw. das Fenster 7, durch welches die Bildaufzeichnung und Beleuchtung des Operationsfelds durch ein Mikroskop erfolgt, wenn der Adapter auf dem Objektiv des Mikroskops montiert ist und weist an seinem anderen (oberen) Ende den die Öffnung 10 des zylindrischen Grundkörpers 6 umlaufenden Flansch/Kragen 8 auf.

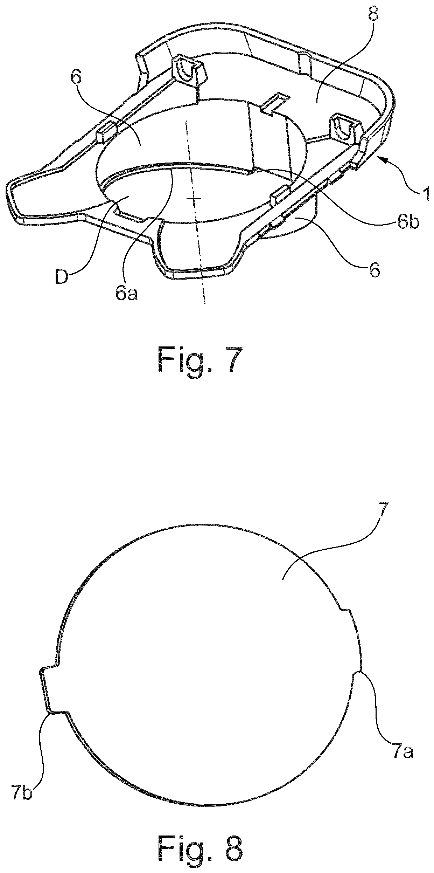

Alternativ zu dieser Ausführung zeigen die Fig. 7 und 8 einen mehrteiligen Aufbau von Grundplatte 1 und Drapelinse 7, d.h. eine Trennung/Aufteilung der Grundplatte 1 in eine separate Scheibe/Drapelinse 7 mit der Fensterfunktion, der Grundplatte 1 mit Stutzen 6 und Kragen 8, sowie einer Fassung, welche die Drapelinse mit der Grundplatte 1 verspannt. Die Fassung kann dabei durch die Kappe 2 gemäß der vorstehenden Ausführungsform oder durch ein weiteres zusätzliches Zwischenbauteil (nicht gezeigt) gebildet sein.

Im Konkreten hat die separate Drapelinse 7 einen kreisförmigen Fensterabschnitt etwa mit Durchmesser entsprechend dem Stutzen-Innendurchmesser, an dessen Außenumfang zwei diametral angeordnete Laschen 7a, 7b angeformt sind, die sich radial nach Außen erstrecken. Der Stutzen 6 der Grundplatte 1 bildet an seinem einen (unteren) Ende/Endabschnitt einen radial nach innen gerichteten Vorsprung/Umfangsring 6a, der als Auflager für die separate Drapelinse 7 dient und in einem Neigungswinkel bezüglich des Kragens/Flanschs 8 bzw. bezüglich der in Fig. 7 angedeuteten Stutzenachse gemäß vorstehender Definition ausgerichtet ist. Zusätzlich ist an der Stutzenwand unmittelbar oberhalb des nach innen gerichteten Vorsprungs/Umfangrings 6a sowie an einem zum Kragen/Flansch 8 am axial weitesten entfernten Umfangsabschnitt des Stutzens 6 ein Ausbruch 6b ausgebildet, der so dimensioniert ist, dass einer der Laschen 7a an der Drapelinse 7 darin eingeführt werden kann, wenn die Drapelinse 7 am nach innen gerichteten Vorsprung/Umfangsring aufliegt. Die diametral gegenüberliegende Lasche 7b ist so dimensioniert, dass sie im Wesentlichen bis zur Oberseite des Kragens/Flanschs 8 reicht, wenn die Drapelinse 7 am nach innen gerichteten Vorsprung/Umfangsring 6a aufliegt. Wird daraufhin die Kappe 2 auf die Oberseite des Kragens/Flanschs 8 der Grundplatte 1 aufgesetzt und dort fixiert/verclippt, drückt die Kappe 2 zwangsläufig auf die eine obere Lasche 7b und verspannt so die Drapelinse 7 zwischen sich und dem nach innen gerichteten Vorsprung/Umfangsring 6a.

In der Figur 3a ist des Weiteren gezeigt, dass gemäß der vorliegenden Ausführungsform die Grundplatte 1 zwei Begrenzungselemente 5 bzw. Anschläge / Vorsprünge aufweist, die jeweils auf einander entgegengesetzten Seiten der Öffnung 10 des zylindrischen Körpers der Grundplatte 1 (diametral gegenüberliegend) vorgesehen sind und eine Bewegung des beweglichen Elements 3 aus der Offenstellung in die Arretierungsstellung begrenzen.

Auf der in Figur 3a links gezeigten Randseite 12 weist die Grundplatte 1 einen Teil bzw. Teilbereich 11 der Ausrichtungsaufnahme 4 auf, welcher später mit einem bestimmten Teilbereich 16 der Kappe 2 zusammenwirkt, um so die Ausrichtungsaufnahme 4 zu bilden. An dieser Randseite 12 ist ferner ein umlaufender Rahmen angeformt, der sich senkrecht zum Flansch/Kragen ausrichtet und diesen aussteift. In diesen Rahmen ist eine co-axial zum Stutzen sich erstreckende innere Kerbe eingearbeitet, welche den einen Teil/Teilbereich 11 der Ausrichtungsaufnahme 4 bildet.

Der in der Figur 3a links gezeigte Bereich der Grundplatte 1, in welchem sich auch die Ausrichtungsaufnahme 4 bzw. zumindest der von der Grundplatte 1 ausgebildete Teil bzw. Teilbereich 11 der Ausrichtungsaufnahme 4 befindet, bildet die Randseite 12 einen im Wesentlichen geraden Randabschnitt mit abgerundeten Ecken 13, wobei ein Benutzer bei der Montage des Adapters an dem Mikroskop seine Finger (z.B. Zeigefinger, Mittelfinger, Ringfinger) an der Randseite 12 anlegen kann, um somit ein Widerlager zu haben, um Druck auf den Betätigungsabschnitt 9 des beweglichen Elements 3 auszuüben, um so den Adapter in Offenstellung zu bringen oder zu halten.

Auf der der Randseite 12 mit dem Teil bzw. Teilbereich 11 der Ausrichtungsaufnahme 4 gegenüberliegenden Randseite 14 der Grundplatte 1 kann die Grundplatte 1 jeweils an den Ecken laschenartige Vorsprünge 15 aufweisen, wodurch die Kontur der Stirnseite 14 eine Muldenform definiert.

Dies erleichtert das Ausüben von Druck auf den Betätigungsabschnitt 9 des beweglichen Elements 3, der zwischen den beiden laschenartigen Vorsprüngen 15 positioniert ist.

In der Figur 3b ist eine Draufsicht der in Figur 3a gezeigten Grundplatte 1 dargestellt. Dieselben Bezugszeichen beziehen sich auf dieselben Elemente bzw. Elemente.

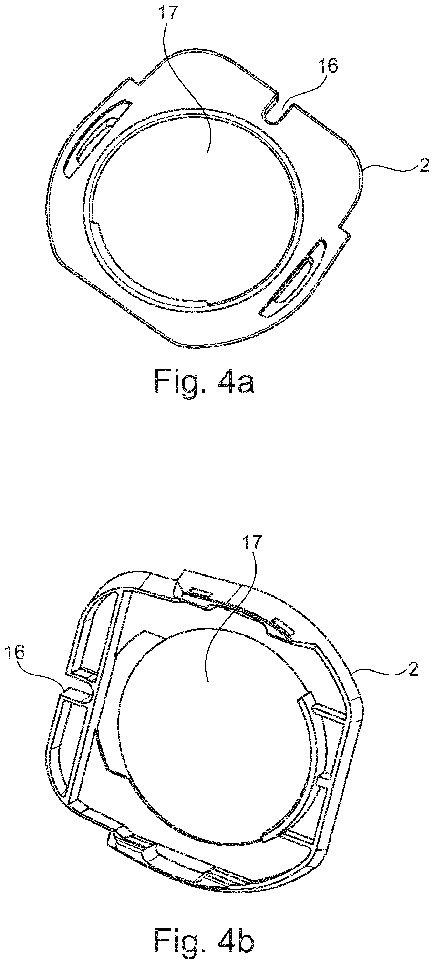

In den Figuren 4a und 4b ist die Kappe 2 des erfindungsgemäßen Adapters in Draufsicht und in einer Ansicht von unten zu sehen.

Wie in der Figur 4a ersichtlich, bildet die Kappe 2 in einem Abschnitt einen Teil bzw. Teilbereich 16 der Ausrichtungsaufnahme 4 aus. Dieser Teil bzw. Teilbereich 16 wirkt mit dem vorstehend beschriebenen Teil bzw. Teilbereich 11 der Grundplatte 1 so zusammen, dass diese Teile bzw. Teilbereiche 11, 16 gemeinsam die Ausrichtungsaufnahme 4 ausbilden.

In den Figuren 5a und 5b ist das bewegliche Element (Zungenplatte) 3 des erfindungsgemäßen Adapters zu sehen.

Das bewegliche Element 3 ist ein Bauteil, welches im Wesentlichen die Form eines Ringes/Ringscheibe hat. Bei dem in den Figuren 5a und 5b gezeigten beweglichen Element 3 ist die Öffnung 18 bzw. der Durchmesser der Öffnung 18 des beweglichen Elements 3 größer als die Öffnungen 10, 17 bzw. der Durchmesser der Öffnungen 10, 17 des zylindrischen Körpers/Stutzens 6 der Grundplatte 1 und der Kappe 2, welche in den Figuren 3a bis 3b und 4a bis 4b gezeigt sind.

Zudem ist in den Figuren 5a und 5b gezeigt, dass die Öffnung 18 des beweglichen Elements 3 nicht exakt kreisförmig ist, jedoch gekrümmte Abschnitte 18a, 18b aufweist, die jeweils einem Kreisausschnitt entsprechen (wodurch eine Art Ellipsenform entsteht). Wie in den Figuren 5a und 5b gezeigt, ist zumindest der Kreisausschnitt 18b, welcher die Kontur der Öffnung 18 des beweglichen Elements 3 auf der Seite bestimmt, welche dem Betätigungsabschnitt 9 nächstgelegen ist, ein Kreisausschnitt eines Kreises, dessen Radius größer ist als der Radius des Kreises, welcher den Öffnungen 10, 17 des zylindrischen Körpers 6 der Grundplatte 1 und der Kappe 2 zugrunde liegt.

In anderen Worten ist der dem Betätigungsabschnitt 9 des beweglichen Elements 3 nächstliegende Kreisausschnitt 18b, welcher die Kontur der Öffnung 18 des beweglichen Elements 3 bildet, weniger gekrümmt als der Kreis, welcher den Öffnungen 10,17 des zylindrischen Körpers 6 der Grundplatte 1 und der Kappe 2 zugrunde liegt.

Zudem weist das bewegliche Element 3, wie in den Figuren 5a und 5b gezeigt, zwei in Element-Ebenenrichtung sich erstreckende Haltestifte 19 für Vorspannelemente, insbesondere in Form von Schraubenfedern bzw. Druckfedern, auf.

Wird der erfindungsgemäße Adapter zusammengesetzt, bzw. wird das bewegliche Element 3 auf die Grundplatte 1, wie sie beispielsweise in den Figuren 3a und 3b gezeigt ist, aufgelegt bzw. aufgebracht, so werden die Vorspannelemente / Druckfedern / Schraubenfedern zwischen den Haltestiften 19 des beweglichen Elements 3 und entsprechenden gegenlagern der Grundplatte 1 und / oder der Kappe 2, um so das bewegliche Element 3 in die Arretierungsposition bzw. Arretierungsstellung vorzuspannen.

Die vorliegende Erfindung betrifft nochmals zusammenfassend einen Montageadapter zur lösbaren Befestigung eines Sterilüberzugs an einem Objektiv eines Mikroskops, mit einem Durchgang D für das darin Einführen des Objektivs, wobei in dem Durchgang eine Drapelinse oder Licht-transparente Scheibe 7 angeordnet ist, derart, dass diese einen schiefen Winkel zur Längsachse des Durchgangs D einnimmt und mit einer manuell betätigbaren Rasteinrichtung 3 für das axiale Halten des Montageadapters am in den Durchgang D eingeführten Objektiv. Erfindungsgemäß ist eine Positioniergeometrie 4 vorgesehen, die dafür ausgebildet und angeordnet ist, eine reproduzierbare Soll-Winkelpositionierung oder Soll-Rotationsausrichtung des Montageadapters am Objektiv ausschließlich durch dessen axiales Aufstecken zu bewirken und unabhängig und separat zur Rastvorrichtung ihre Positionierfunktion zu erfüllen.

## Patentansprüche

1. Montageadapter zur lösbaren Befestigung eines Sterilüberzugs an einem Objektiv eines Mikroskops, mit einem Durchgang (D) für das darin Einführen des Objektivs, in welchem eine Drapelinse oder Licht-transparente Scheibe (7) angeordnet ist, derart, dass diese einen schiefen Winkel zur Längsachse des Durchgangs (D) einnimmt und mit einer manuell betätigbaren Rasteinrichtung (3) für das axiale Halten des Montageadapters am in den Durchgang (D) eingeführten Objektiv, und eine Positioniergeometrie (4), die dafür ausgebildet und angeordnet ist, eine reproduzierbare Soll-Winkelpositionierung und/oder Soll- Rotationsausrichtung des Montageadapters am Objektiv ausschließlich durch dessen axiales Aufstecken zu bewirken und unabhängig und separat zur Rastvorrichtung ihre Positionierfunktion zu erfüllen.

2. Montageadapter nach Anspruch 1, **dadurch gekennzeichnet, dass** die Positioniergeometrie (4) derart ausgebildet und/oder am Montageadapter angeordnet ist, dass diese bei einer Ist-Winkelposition ungleich der Soll-Winkelposition ein Einführen des Objektivs in den Durchgang (D) bis zu einer solchen Soll-Einführtiefe verhindert, in welcher die Rasteinrichtung (3) ein axiales Halten des Montageadapters am Objektiv bewirkt.

3. Montageadapter nach Anspruch 1 oder 2 **dadurch gekennzeichnet, dass** die Positioniergeometrie (4) in Form einer Asymmetrie insbesondere in/an dem Durchgang (D) oder in Form einer Ausrichtungsaufnahme, insbesondere einer Ausnehmung oder eines Rücksprungs vorzugsweise mit axial wirkendem Endanschlag oder eines Vorsprungs am Montageadapter sowie weiter vorzugsweise im Radialabstand zum Durchgang (D) ausgebildet ist.

4. Montageadapter nach Anspruch 3, **dadurch gekennzeichnet, dass** die Ausrichtaufnahme ein Sackloch ist, das sich co-axial sowie im Radialabstand zum Durchgang (D) in den Montageadapter erstreckt.

5. Montageadapter nach einem der vorstehenden Ansprüche, **gekennzeichnet durch** eine Grundplatte (1) bestehend aus einem eine Durchgangsöffnung bildenden Stutzen (6), an dessen einem axialen Endabschnitt die Drapelinse oder Licht-durchlässige Scheibe (7) angeordnet ist und dessen anderer axialer Endabschnitt von einem Flansch (8) umgriffen ist, auf dessen Stutzenabgewandter Seite eine Kappe (2) mit Durchgangsöffnung aufgesetzt ist, derart, dass die Durchgangsöffnungen der Grundplatte (1) und der Kappe (2) unter Ausbildung des gemeinsamen Durchgangs (D) fluchten und sich dazwischen ein Aufnahmefach ausbildet, in das eine manuell bewegbare Zungenplatte (3) für ein wahlweises Einengen des Durchgangs (D) eingeführt ist.

6. Montageadapter nach Anspruch 5, **dadurch gekennzeichnet, dass** die Drapelinse oder Licht-transparente Scheibe (7) als ein zur Grundplatte (1) und zur Kappe (2) separates Bauteil ausgebildet ist und im Stutzen (6) der Grundplatte (1) ein radial nach innen vorragender Absatz (6a) ausgeformt oder angeordnet ist, der eine axiale Auflage für die Dapelinse oder Licht-transparente Scheibe (7) bildet.

7. Montageadapter nach Anspruch 6, **dadurch gekennzeichnet, dass** die Kappe (2) an ihrer, der Grundplatte (1) zugewandten Seite ein Andrückmittel oder einen Adrückabschnitt hat, der bei Aufsetzen der Kappe (2) auf die Grundplatte (1) die Drapelinse oder Licht-durchlässige Scheibe (7) zwischen sich und der Stutzen-seitigen axialen Auflage (6a) einspannt.

8. Montageadapter nach Anspruch 7, **dadurch gekennzeichnet, dass** der Stutzen (6) mit einem Ausbruch oder Rücksprung (6b) ausgebildet ist und die Drapelinse oder Licht-durchlässige Scheibe (7) eine radial vorragende Lasche aufweist, die in den Ausbruch oder Rücksprung für ein axiales Halten der Drapelinse oder Licht-durchlässige Scheibe (7) eingeführt ist.

9. Mikroskop, mit einem Objektiv, welches für eine Verwendung mit einem Montageadapter gemäß einem der Ansprüche 1 bis 8 ausgelegt ist, wobei das Mikroskop einen Eingreifabschnitt vorzugsweise in Form eines co-axial zum Objektiv sich erstreckenden Ausrichtungszapfens aufweist, welcher dazu ausgelegt ist, in die Ausrichtungsaufnahme (4) des Adapters eingeführt zu werden und der dafür vorgesehen und ausgebildet ist, im Zusammenwirken mit der Ausrichtungsaufnahme (4) zumindest eine bestimmte Soll-Winkelposition des Montageadapters und eine bestimmte Soll-Axialposition des Montageadapters am Objektiv festzulegen.

10. System aus einem Montageadapter nach einem der Ansprüche 1 bis 8 und einem Mikroskop gemäß Anspruch 9.

## Claims

1. Mounting adapter for releasably fastening a sterile cover to an objective of a microscope, comprising a passage (D) for inserting the objective therein, in which a drape lens or light-transparent disc (7) is arranged such that the latter assumes an oblique angle in relation to the longitudinal axis of the passage (D), and comprising a manually actuable latching device (3) for axially holding the mounting adapter on the objective inserted into the passage (D), and a positioning geometry (4) which is configured and arranged to cause a reproducible target angular positioning and/or target rotational alignment of the mounting adapter on the objective exclusively by its axial fitting and to perform its positioning function independently and separately from the latching apparatus.

2. Mounting adapter according to Claim 1, **characterized in that** the positioning geometry (4) is configured and/or arranged on the mounting adapter in such a way that, when an actual angular position is unequal to the target angular position, the positioning geometry prevents the objective being inserted into the passage (D) to such a target insertion depth at which the latching device (3) causes the mounting adapter to be held axially on the objective.

3. Mounting adapter according to Claim 1 or 2, **characterized in that** the positioning geometry (4) is configured in the form of an asymmetry in particular in/on the passage (D) or in the form of an alignment receptacle, in particular of a clearance or of a recess preferably with axially acting end stop or of a projection on the mounting adapter and further preferably at a radial distance from the passage (D).

4. Mounting adapter according to Claim 3, **characterized in that** the alignment receptacle is a blind hole which extends into the mounting adapter coaxially and at a radial distance from the passage (D).

5. Mounting adapter according to one of the preceding claims, **characterized by** a base plate (1) comprising a socket (6) which forms a passage opening and at one axial end portion of which the drape lens or light-permeable disc (7) is arranged and the other axial end portion of which is surrounded by a flange (8), on the side facing away from the socket of which a cap (2) with passage opening is fitted such that the passage openings of the base plate (1) and of the cap (2) are aligned, forming the common passage (D), and a receiving compartment is formed therebetween, into which a manually movable tongue plate (3) is inserted for selectively narrowing the passage (D).

6. Mounting adapter according to Claim 5, **characterized in that** the drape lens or light-transparent disc (7) is configured as a component separate from the base plate (1) and from the cap (2), and a radially inwardly projecting shoulder (6a), which forms an axial support for the drape lens or light-transparent disc (7), is formed or arranged in the socket (6) of the base plate (1).

7. Mounting adapter according to Claim 6, **characterized in that** the cap (2) has on its side facing the base plate (1) a pressing means or a pressing portion which, when the cap (2) is fitted on the base plate (1), clamps the drape lens or light-permeable disc (7) between itself and the socket-side axial support (6a) .

8. Mounting adapter according to Claim 7, **characterized in that** the socket (6) is configured with a cutout or recess (6b), and the drape lens or light-permeable disc (7) has a radially projecting lug which is inserted into the cutout or recess for axially holding the drape lens or light-permeable disc (7).

9. Microscope having an objective which is configured for use with a mounting adapter according to one of Claims 1 to 8, wherein the microscope has an engagement portion preferably in the form of an alignment pin which extends coaxially to the objective and which is designed to be inserted into the alignment receptacle (4) of the adapter and which is provided and configured to determine, in cooperation with the alignment receptacle (4), at least one specific target angular position of the mounting adapter and a specific target axial position of the mounting adapter on the objective.

10. System consisting of a mounting adapter according to one of Claims 1 to 8 and a microscope according to Claim 9.

## Revendications

1. Adaptateur de montage pour la fixation amovible d'une housse stérile sur l'objectif d'un microscope, avec un passage (D) pour l'introduction de l'objectif en lui, une lentille drapée ou un disque (7) transparent à la lumière étant agencé(e) dans le passage de telle sorte que cette dernière/ce dernier forme un angle oblique par rapport à l'axe longitudinal du passage (D) et comprenant un dispositif de verrouillage (3) actionnable manuellement pour le maintien axial de l'adaptateur de montage sur l'objectif introduit dans le passage (D), et une géométrie de positionnement (4) qui est conçue et agencée de façon à assurer un positionnement angulaire cible et/ou une orientation en rotation cible reproductibles de l'adaptateur de montage sur l'objectif exclusivement par son enclenchement axial et pour remplir sa fonction de positionnement indépendamment et séparément du dispositif de verrouillage.

2. Adaptateur de montage selon la revendication 1, **caractérisé en ce que** la géométrie de positionnement (4) est conçue et/ou agencée sur l'adaptateur de montage de telle sorte que, dans le cas d'une position angulaire réelle différente de la position angulaire cible, elle empêche l'introduction de l'objectif dans le passage (D) jusqu'à une profondeur d'introduction cible dans laquelle le dispositif de verrouillage (3) assure un maintien axial de l'adaptateur de montage sur l'objectif.

3. Adaptateur de montage selon la revendication 1 ou 2, **caractérisé en ce que** la géométrie de positionnement (4) est réalisée sous la forme d'une asymétrie, en particulier dans/sur le passage (D), ou sous la forme d'un logement d'alignement, en particulier d'un évidement ou d'un retrait, de préférence avec une butée d'extrémité agissant axialement, ou d'une saillie sur l'adaptateur de montage, ainsi que, de préférence, d'une distance radiale du passage (D).

4. Adaptateur de montage selon la revendication 3, **caractérisé en ce que** le logement d'alignement est un trou borgne qui s'étend dans l'adaptateur de montage, coaxialement ainsi qu'à une distance radiale du passage (D) .

5. Adaptateur de montage selon l'une des revendications précédentes, **caractérisé par** une plaque de base (1) constituée d'un manchon (6) formant une ouverture de passage, sur une section d'extrémité axiale duquel est agencée la lentille drapée ou le disque (7) transparent à la lumière et dont l'autre section d'extrémité axiale est entourée par une bride (8), une pièce de recouvrement (2) étant placée sur le côté opposé au manchon avec une ouverture de passage, de telle sorte que les ouvertures de passage de la plaque de base (1) et de la pièce de recouvrement (2) s'alignent en formant le passage commun (D) et qu'il se forme entre elles un compartiment de réception dans lequel est introduite une plaque à languette (3) déplaçable manuellement pour, de façon optionnelle, rétrécir le passage (D).

6. Adaptateur de montage selon la revendication 5, **caractérisé en ce que** la lentille à drapage ou le disque (7) transparent à la lumière est conçu sous la forme d'un composant séparé de la plaque de base (1) et de la pièce de recouvrement (2), et **en ce que** dans le manchon (6) de la plaque de base (1) est formé ou agencé un épaulement (6a) faisant saillie radialement vers l'intérieur, qui forme un appui axial pour la lentille drapée ou le disque (7) transparent à la lumière.

7. Adaptateur de montage selon la revendication 6, **caractérisé en ce que** la pièce de recouvrement (2) comporte, sur son côté tourné vers la plaque de base (1), un moyen de pression ou une section de pression qui, lors de la mise en place de la pièce de recouvrement (2) sur la plaque de base (1), serre la lentille drapée ou le disque (7) transparent à la lumière entre lui-même et l'appui axial (6a) situé du côté du manchon.

8. Adaptateur de montage selon la revendication 7, **caractérisé en ce que** le manchon (6) est réalisé avec une découpe ou un retrait (6b) et **en ce que** la lentille drapée ou le disque (7) transparent à la lumière présente une languette faisant saillie radialement, qui est insérée dans la découpe ou le retrait pour un maintien axial de la lentille drapée ou du disque (7) transparent à la lumière.

9. Microscope comprenant un objectif conçu pour être utilisé avec un adaptateur de montage selon l'une quelconque des revendications 1 à 8, le microscope comprenant une partie d'engagement, de préférence sous la forme d'une broche d'alignement s'étendant coaxialement à l'objectif, qui est conçue pour être insérée dans le logement d'alignement (4) de l'adaptateur et qui est prévue et configurée pour définir, en coopération avec le logement d'alignement (4), au moins une position angulaire cible déterminée de l'adaptateur de montage et une position axiale cible déterminée de l'adaptateur de montage sur l'objectif.

10. Système constitué d'un adaptateur de montage selon l'une des revendications 1 à 8 et d'un microscope selon la revendication 9.
